Europäisches Patentamt

European Patent Office

Office européen des brevets

(19)

(11) Publication number: **0 054 296**
**B1**

(12) **EUROPEAN PATENT SPECIFICATION**

(45) Date of publication of patent specification: **03.07.85**

(51) Int. Cl.⁴: **A 61 B 17/36**

(21) Application number: **81110431.4**

(22) Date of filing: **14.12.81**

(54) Attachment for laser device.

(30) Priority: **15.12.80 JP 178744/80 U**
**15.12.80 JP 178745/80 U**

(43) Date of publication of application:
**23.06.82 Bulletin 82/25**

(45) Publication of the grant of the patent:
**03.07.85 Bulletin 85/27**

(84) Designated Contracting States:
**DE FR GB SE**

(56) References cited:
**FR-A-2 202 674**

**Mechanism, Linkages and Mechanical
Controls, 1965, edited by Nicholas P. Chironis,
McGraw Hill Book Co., p. 346 and 347**

(73) Proprietor: **SUMITOMO ELECTRIC INDUSTRIES
LIMITED**
**No. 15, Kitahama 5-chome Higashi-ku
Osaka-shi Osaka-fu (JP)**

(72) Inventor: **Ono, Kimizo Sumitomo Electric Ind.,
Ltd.**
**Osaka Works, No. 1-3, Shimaya 1-chome
Konohana-ku Osaka-shi Osaka (JP)**
Inventor: **Sato, Koji Sumitomo Electric Ind., Ltd.**
**Osaka Works, No. 1-3, Shimaya 1-chome
Konohana-ku Osaka-shi Osaka (JP)**
Inventor: **Takenaka, Shinya Sumitomo Electric
Ind., Ltd.**
**Osaka Works, No.1-3, Shimaya 1-chome
Konohana-ku Osaka-shi Osaka (JP)**
Inventor: **Sunago, Katsuyoshi Sumitomo
Electric Ind., Ltd.**
**Osaka Works, No. 1-3, Shimaya 1-chome
Konohana-ku Osaka-shi Osaka (JP)**
Inventor: **Oka, Hidekazu Sumitomo Electric Ind.,
Ltd.**
**Osaka Works, No. 1-3, Shimaya 1-chome
Konohana-ku Osaka-shi Osaka (JP)**

Courier Press, Leamington Spa, England.

**0 054 296**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath Maximilianstrasse 58 D-8000 München 22 (DE)**

## Description

This invention relates to attachment means for attaching first and second parts of a laser device to each other at mating ends thereof. Said parts can be laser machining apparatus or a laser knife which can be disassembled into separate components according to their functions.

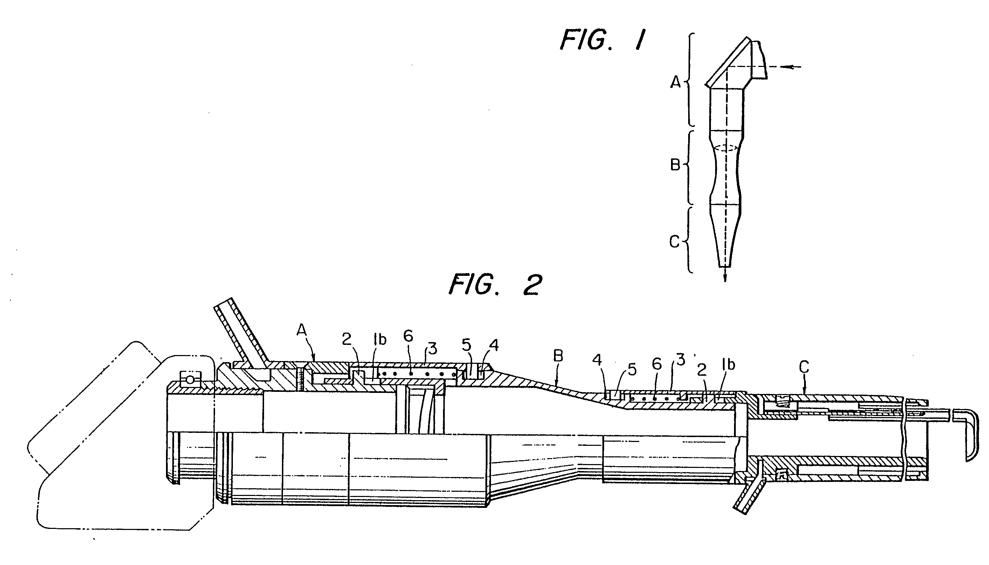

A hand piece unit at the end of a light conducting path for a laser device such as a laser knife, as shown in Fig. 1, comprises: a hand piece section A; a lens holder section B; and an end adaptor section C. Depending on the kinds of surgical operations to be performed, it is necessary to couple focusing lenses of different focal lengths or end adaptors of different configurations to the laser knife. For this purpose, the hand piece unit is divided into the three components mentioned above, and these components are connected together or interchanged by screwing. However, this screwing system is disadvantageous in that, since each component must be turned at least several turns until it is connected or disconnected, it takes an undesirably long time to exchange the lens holder section B or the end adaptor section C, and accordingly it is impossible to reduce the time required for a surgical operation.

Another possibility to attach the respective parts together is described by the FR—A—2202674. According to this known prior art, the two respective parts are put together, without rotating one part against the other part and without securing said parts against such a rotation.

Accordingly, an object of this invention is to provide an attachment means for a laser device, by which the components of the laser device can also be readily and quickly connected to and disconnected from each other and by which they are secured and fixed against each other in the connected operation mode. The device should even be simple in construction.

The foregoing objects and other objects of the invention have been achieved by the provision of an attachment means for attaching first and second parts of the laser device to one another at mating ends thereof with an engaging groove formed in said first part, said engaging groove comprising an inserting groove portion opening to the mating end of the first part and extending in a first direction away from said mating end and a locking groove portion contiguous with the inserting groove portion and extending in a second direction different from said first direction, with a pin protruding from the second part and insertable into the engaging groove, with holding means for holding the pin in the locking groove portion, whereby said holding means comprise a cover member slidably mounted on the first part for movement axially with respect of said first part and spring biased toward the mating end of the first part from a first position in which the pin is freely movable in the locking groove portion to a second position in which it engages the pin for holding it in said locking groove portion.

The nature, principle and utility of the invention will become more apparent from the following detailed description when read in conjunction with the accompanying drawings, in which:

Fig. 1 is a front view showing the arrangement of the hand piece unit of a laser knife;

Fig. 2 is a longitudinal sectional view showing the connecting arrangement of the hand piece unit of a laser knife which employs an attachment according to a first embodiment of this invention;

Fig. 3 is a perspective view showing the essential components of the attachment;

Fig. 4 is a longitudinal sectional view showing the connecting arrangement of the lens holder section and the end adaptor section of a laser knife which employs an attachment according to a second embodiment of the invention; and

Fig. 5 is a perspective view showing the essential components of the attachment in Fig. 4.

Detailed Description of the Invention

Preferred embodiments of this invention will be described by way of example with reference to attachments used in the hand piece unit of a laser knife.

As described above, a hand piece unit at the end of the light conducting path of a laser knife comprises a hand piece articulation section A, a lens holder section B, and an end adaptor section C. These connecting parts A, B and C are connected together through attachments formed at the ends thereof. Each attachment comprises: an engaging groove 1; an engaging pin 2 which is engaged with the engaging groove 1; and a cover 3 used to push back these connecting components.

In a first embodiment of the invention shown in Fig. 2, in order to join the hand piece articulation section A and the lens holder section B together, the hand piece articulation section A has an engaging pin 2 while the lens holder section B has the engaging groove 1 and the cover; and in order to join the lens holder section B and the end adaptor section C together, the lens holder section B has an engaging pin 2 and the cover 3 while the end adaptor section C has the engaging groove. It is desirable to provide at least two pairs of engaging grooves 1 and engaging pins 2 so that the axis of the light conducting path is maintained correct. However, one pair of an engaging groove 1 and engaging pin is sufficient if the connecting ends are formed accurately.

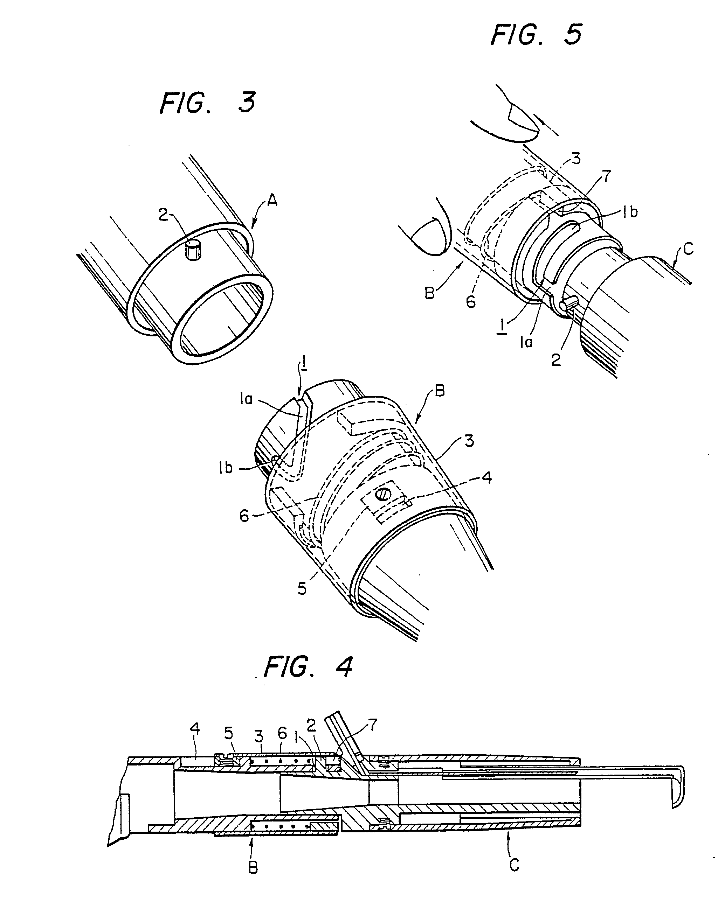

The engaging groove 1, as shown in Fig. 3, comprises an inserting groove 1a which extends from the end face of the connecting part inwardly thereof, and a locking groove 1b which extends from the inserting groove 1a outwardly of the connecting part, i.e., towards the end face of the connecting part, so as to stop the circumferential movement of the engaging pin provided on the mating connecting part. In this embodiment, the inserting groove 1a of the engaging groove 1 is inclined with respect to the axis of the connecting parts; however, it may instead be extended in parallel with the axis. In other words, all that is necessary for the engaging groove 1 is that the inserting groove 1a extend from the end face of the connecting part inwardly thereof and the locking groove 1b extend from the inserted groove at least slightly in the opposite direction. In the case where the engaging groove 1 is L-shaped, it is necessary to push and turn one of the connecting parts. In the case of the illustrated embodiment, it is not necessary to push, but the engaging pin 2 can be engaged with the locking groove 1b merely by turning the connecting parts relative to each other.

The engaging pin 2 is formed on the outer wall of the hand piece articulation section A or the lens holder section B in such a manner that it protrudes radially outwardly thereof. More specifically, the engaging pin 2 is positioned in correspondence to the position of the engaging groove 1 and over the turning point of the groove 1.

The lens holder section B has a cover 3 which is used to push back the connecting parts which have been joined together by engaging the engaging pin 2 with the engaging groove 1, i.e. it urges the parts in opposite directions to secure the pin in the groove 1b. The cover 3 is so designed that it is not turned and is moved only in the axial direction by means of a sliding key 5 set in a guide groove 4 which is formed in the lens holder section B. The cover 3 incorporates a spring 6 so that the mating connecting parts, namely the hand piece articulation section A and the end adaptor section C which have been pushed into one another, are pushed back by the spring.

If, after the engaging pin 2 of the hand piece articulation section A has been set in the inserting groove 1a of the engaging groove 1 of the lens holder section B, the lens holder section B is turned relative to the section A while being pushed in, then the engaging pin 2 will follow the engaging groove 1 while the cover 3 is being pushed by the end face of the hand piece articulation section, and finally the engaging pin 2 will be set in the locking groove 1b. The hand piece articulation section A and the lens holder section B, while being pushed apart by the action of the cover 3, are positively coupled to each other by the engagement of the engaging pin 2 and the locking groove 1b. That is, the sections A and B are positively connected when the engaging pin 2 is pushed to the end of the engaging groove 1. The hand piece articulation section A is disconnected from the lens holder section B as follows. The hand piece articulation section A is pushed towards the lens holder section B and turned to disengage the engaging pin 2 from the locking groove 1b, and the hand piece articulation section A is then pulled to move the engaging pin 2 along the inserting groove 1a.

The end adaptor section C is connected to and disconnected from the lens holder section B similarly as in the above-described case.

In the above-described embodiment, the engaging pins 2 are provided on the hand piece articulation section A and the end adaptor section C; however, the engaging pins 2 may be provided instead on the lens holder section B. Also in the above-described embodiment, the cover 3 is provided on the lens holder section B, but it may obviously be provided on any one of the connecting parts. Furthermore, the connecting pin 2 may be provided on the inner wall of one of the connecting parts instead of the outer wall.

A second embodiment of the invention is shown in Figs. 4 and 5. An attachment, as shown in Fig. 4, comprises an engaging groove 1, an engaging pin 2 which is engaged with the engaging groove 1, and a cover 3 for fixing the engaging pin 2. The attachment is used to couple the lens holder section B to the end adaptor section C, for example.

The lens holder section B has the engaging groove 1, while the end adaptor section C has the engaging pin 2. It is desirable that at least two pairs of engaging grooves 1 and engaging pins 2 are provided symmetrically so that the axis of the light conducting path is maintained correct, but one pair may be sufficient if the connecting ends are formed accurately similarly as in the case of the first embodiment.

The engaging groove 1, as shown in Fig. 5, is a bent groove which consists of an inserting groove 1a which is extended from the inserting groove 1a circumferentially of the connecting part, to stop the axial movement of the engaging pin 2. In this embodiment, the inserting groove 1a extended axially is perpendicular to the locking groove 1b extended circumferentially. However, the engaging groove 1 may be so modified that the inserting groove 1a is inclined with respect to the axis of the lens holder section B, and the locking groove 1b is provided at the end of the inserting groove 1a thus inclined. In this modification, a force of pushing the end adaptor section C with the engaging pin 2 into the lens holder section B can be obtained by rotation of the end adaptor section C, and therefore the two sections B and C can be readily connected to each other.

The engaging pin 2 is formed on the outer wall of the end adaptor section C in such a manner that it protrudes radially outwardly of the section C. More specifically, the engaging pin 2 is positioned in agreement with the position of the locking groove 1b of the engaging groove 1, so that when the pin 2 is set in the locking groove 1b, the distance between the end adaptor section C and the lens holder section B is set to a desired value.

The lens holder section B has the cover 3 which is adapted to stop the rotation of the engaging pin 2 set in the locking groove 1b, as was described above. The cover 3 is so designed that it is not turned and is movable only in the axial direction by a sliding key 5 which is set in a guide groove 4 which is formed in the lens holder section B. A spring 6 is interposed between the cover 3 and the lens holder section B, so as to push the cover 3 towards the end adaptor section C. The cover 3 has an engaging groove 7 which surrounds the engaging pin 2 of the end adaptor section C, to prevent the engaging pin 2 from being turned circumferentially of the section C.

If, after the engaging pin 2 of the end adaptor section C is set in the inserting groove 1a of the engaging groove 1 of the lens holder section B, the end adaptor section C is pushed and turned relative to the lens holder section B, the cover 3 pushed by the engaging pin 2 is caused to spring towards the end adaptor section C by the elastic force of the spring 6 when the pin 2 aligns with the engaging groove 7 of the cover 3, as a result of which the engaging pin 2 is surrounded by the engaging groove 7, i.e., it is set in the engaging groove 7. Thus, the engaging pin 2 can move no further in either the circumferential direction or the axial direction; that is, the lens holder section B is fixedly coupled to the end adaptor section C.

The lens holder section B can be disconnected from the end adaptor section C as follows. The cover 3 is pulled towards the lens holder section B so that the engaging pin 2 is movable in the circumferential direction. Then, the end adaptor section C is turned relative to the lens holder section B, to move the engaging pin 2 in the circumferential direction, and then the end adaptor section C is pulled out to remove the engaging pin 2 from the inserting groove 1b.

In the above-described embodiment, the engaging pin 2 is provided on the side of the end adaptor section C; however, the attachment may be so modified that the lens holder section B has the engaging pin 2, while the end adaptor section C has the engaging groove 1 and the cover 3. Furthermore, the engaging pin 2 may protrude from the inside wall of the connecting part, instead of the outer wall.

As is apparent from the above description, in a laser device made up of separable connecting parts according to this invention, one of the connecting parts has an engaging pin, and its mating connecting part has both an engaging groove for preventing the circumferential and axial movements of the engaging pin and a cover for moving the engaging pin in a direction opposite to the direction in which the engaging pin has been moved in engagement. Therefore, if, after the engaging pin of the one connecting part has been aligned with the engaging groove of the mating connecting part, the two connecting parts are turned relative to each other, than the engaging pin is automatically fixedly set in the engaging groove or in the end of the locking part thereof. That is, the connecting parts of the laser device can be connected by one operation wherein they are turned relative to each other while being pushed in. The connecting parts can be also readily disconnected. That is, the disconnection can be achieved by turning one of the connecting parts to move the engaging pin from the locking groove to the inserting groove or by turning the connecting parts relative to each other after the cover has been pulled in.

Thus, in the case of a laser knife consisting of three separable parts, depending on surgical operation methods or the body parts to be operated on, the lens holder section or the end adaptor section can be readily exchanged, which will contribute to a reduction of the time required for a surgical operation. The attachment is considerably simple in arrangement because it utilizes the engagement of the pin and the groove.

**Claims**

1. An attachment means for attaching first and second parts (B, A) of a laser device to one another at mating ends thereof, characterised in that an engaging groove (1) is formed in said first part, said engaging groove comprising an inserting groove portion (1a) opening to the mating end of the first part and extending in a first direction away from said mating end, and a locking groove portion (1b) contiguous with the inserting groove portion and extending in a second direction different from said first direction, and that a pin (2) is provided protruding from the second part and insertable into the engaging groove and that holding means for holding the pin in the locking groove portion are provided, whereby the holding means comprise a cover member (3) slidably mounted on the first part for movement axially with respect of said first part and spring biased (6) toward the mating end of the first part from a first position in which the pin is freely movable in the locking groove portion to a second position in which it engages the pin for holding it in said locking groove portion.

2. An attachment means according to claim 1, characterised in that the locking groove portion (1b) extends from the inserting groove portion (1a) circumferentially about said first part (B) in a plane substantially perpendicular to a central axis of the first part and the cover member is provided with a rectilinear receiving groove (7) engaging the pin when the cover member is in its second position.

3. An attachment means according to claim 1, characterised in that the locking groove portion (1b) extends from the inserting groove portion (1a) in a direction at least slightly toward the mating end of the first part, and the cover member in its second position urges the pin toward the mating end of the first part.

**Patentansprüche**

1. Verbindungsvorrichtung, um einen ersten und einen zweiten Teil (B, A) einer Lasereinrichtung an Kupplungsenden miteinander zu verbinden, dadurch gekennzeichnet, daß in dem ersten Teil eine Rastnut (1) ausgebildet ist, die einen Einstecknutabschnitt (1a) aufweist, der zum Kupplungsende des ersten Teiles hin geöffnet ist und der sich von diesem Kupplungsende weg in einer ersten Richtung erstreckt und die einen Verschlußnutabschnitt (1b) in Ausrichtung mit dem Einstecknutabschnitt umfaßt, der sich in einer zweiten, von der ersten Richtung unterscheidenden Richtung erstreckt, und daß ein Stift (2) vorgesehen ist, der am zweiten Teil absteht und der in die Rastnut einführbar ist und daß eine Halteeinrichtung vorgesehen ist, um den Stift in dem Verschlußnutabschnitt zu halten, wobei die Halteeinrichtung ein Überwurfelement 3 umfaßt, welches gleitend auf dem ersten Teil befestigt und axial bezüglich des ersten Teils beweglich ist und welches auf das Kupplungsende des ersten Teiles zu aus einer ersten Position, in der der Stift in dem Verschlußnutabschnitt frei beweglich ist auf eine zweite Position zu, in der sie den Stift ergreift, um ihn in dem Verschlußnutabschnitt zu halten, federbeaufschlagt ist.

2. Verbindungsvorrichtung nach Anspruch 1, dadurch gekennzeichnet, daß sich der Verschlußnutabschnitt (1b) von dem Einstecknutabschnitt (1a) weg in Umfangsrichtung über den ersten Teil (B) in einer Ebene erstreckt, die im wesentlichen senkrecht zur Mittelachse des ersten Teils ist und daß das Überwurfelement mit einer rechteckigen Aufnahmenut (7) versehen ist, die mit dem Stift in Eingriff kommt, wenn sich das Überwurfelement in seiner zweiten Position befindet.

3. Verbindungsvorrichtung nach Anspruch 1, dadurch gekennzeichnet, daß sich der Verschlußnutabschnitt (1b) von dem Einstecknutabschnitt (1A) in einer etwas zum Kupplungsende des ersten Teils hin schräg verlaufenden Richtung erstreckt und daß das Überwurf in seiner zweiten Position den Stift auf das Kupplungsende des ersten Teiles hin drückt.

**Revendications**

1. Dispositif de fixation d'une première et d'une seconde partie (B, A) d'un dispositif à laser l'une sur l'autre à des extrémités coopérantes, caractérisé en ce qu'une gorge de coopération (1) est formée dans la première partie, la gorge de coopération ayant une partie de gorge d'introduction (1a) débouchant à l'extrémité de coopération de la première partie et ayant une première direction à partir de l'extrémité de coopération, et une partie de gorge de verrouillage (1b) contiguë à la partie de gorge d'introduction et ayant une seconde direction différente de la première, et en ce qu'un ergot (2) est disposé de manière qu'il dépasse de la seconde partie et qu'il puisse être introduit dans la gorge de coopération, et en ce qu'un dispositif de maintien de l'ergot dans la partie de gorge de verrouillage est disposé, si bien que le dispositif de maintien comporte un organe formant couvercle (3) monté afin qu'il puisse coulisser sur la première partie et se déplace ainsi axialement par rapport à celle-ci, le dispositif de maintien étant rappelé élastiquement (6) vers l'extrémité de coopération de la première partie à partir d'une position dans laquelle l'ergot est mobile librement dans la partie de gorge de verrouillage vers une seconde position dans laquelle il coopère avec l'ergot afin que celui-ci soit maintenu dans la partie de gorge de verrouillage.

2. Dispositif de fixation selon la revendication 1, caractérisé en ce que la partie de gorge de verrouillage (1b) part circonférentiellement à partir de la partie de gorge d'introduction (1a) autour de la première partie (B) dans un plan sensiblement perpendiculaire à un axe central de la première partie, et l'organe formant couvercle a une gorge rectiligne (7) de logement coopérant avec l'ergot lorsque l'organe formant couvercle est dans sa seconde position.

3. Dispositif de fixation selon la revendication 1, caractérisé en ce que la partie de gorge de verrouillage (1b) de la partie de gorge d'introduction (1a) en direction dirigée légèrement vers l'extrémité complémentaire de la première partie, et l'organe formant couvercle, dans sa seconde position, repousse l'ergot vers l'extrémité complémentaire de la première partie.

FIG. 1

FIG. 2

FIG. 3

FIG. 5

FIG. 4